# EUROPÄISCHE PATENTSCHRIFT

(11) **EP 1 094 797 B1**
(45) Veröffentlichungstag und Bekanntmachung des Hinweises auf die Patenterteilung: **06.10.2004**
(21) Anmeldenummer: 99936490.4
(22) Anmeldetag: 07.07.1999
(51) Int. Cl.: A61K 9/70, A61K 31/366

(54) **TRANSDERMALES PFLASTER, ENTHALTEND MINDESTENS EINEN DIE BLUTFETTWERTE BEEINFLUSSENDEN WIRKSTOFF**
TRANSDERMAL PLASTER CONTAINING AT LEAST ONE ACTIVE INGREDIENT WHICH INFLUENCES BLOOD SERUM LIPID LEVELS
EMPLATRE TRANSDERMIQUE CONTENANT AU MOINS UN PRINCIPE ACTIF INFLUANT SUR LE TAUX DE LIPIDES SANGUINS

(30) Priorität: 09.07.1998 DE 19830732
(43) Veröffentlichungstag der Anmeldung: 02.05.2001
(73) Patentinhaber: LTS Lohmann Therapie-Systeme AG, 56626 Andernach (DE)
(72) Erfinder: BERTHOLD, Achim, D-56626 Andernach (DE)
(74) Vertreter: Flaccus, Rolf-Dieter, Dr.
(86) Internationale Anmeldenummer: PCT/EP1999/004757
(87) Internationale Veröffentlichungsnummer: WO 2000/002541

(56) Entgegenhaltungen:
- EP-A- 0 782 861
- WO-A-99/24032
- US-A- 5 629 014

## Beschreibung

Die Erfindung betrifft eine Zubereitung, enthaltend mindestens einen die Blutfettwerte eines Organismus beeinflussenden Wirkstoff.
Bei diesem Wirkstoff handelt es sich um ein Mitglied einer Gruppe von Wirkstoffen, welche in den Lipidstoffwechsel des Organismus eingreifen und zur Behandlung damit in Zusammenhang stehender Erkrankungen eingesetzt werden.
Bei den Stoffen handelt es sich bevorzugt um Inhibitoren der Hydroxy-methyl-Glutaryl-CoA-Reduktase (HMG-CoA-Reduktase).

Systemische Lipidstoffwechselstörungen, insbesondere sogenannte Hyperlipoproteinämien sind in der Pathogenese arteriosklerotischer Gefäßerkrankungen und deren Folgen wie Herzinfarkt, apoplektischer Insult und arterieller Verschlußerkrankungen von großer Bedeutung. In den USA und Europa weisen circa 15 Prozent der Erwachsenen ein erhöhtes Risiko auf, wegen erhöhter Blutlipidwerte kardiovaskuläre Ereignisse zu erleiden. Ein sinnvoller Ansatzpunkt für die Prophylaxe, Therapie und Behandlung der Folgen besteht darin, erhöhte Plasmalipidspiegel zu senken.
Grundlage jeder Behandlung einer Hyperlipoproteinämie ist eine entsprechende Diät. Es muß für eine Gewichtsnormalisierung, für eine geeignete Nahrungszusammensetzung, Fettanteil < 30 % der Gesamtkalorienzahl, für eine ausreichende Ballaststoffzufuhr und für eine reduzierte Cholesterolaufnahme, insbesondere < 300 mg pro Tag gesorgt werden. Ferner ist eine Steigerung der Zufuhr von ungesättigten - vor allem einfach ungesättigten - Fettsäuren zu empfehlen, da diese die Metabolisierung von Lipoproteinen verbessern.
Ist mit den diätetischen Maßnahmen allein keine ausreichende Normalisierung des Lipidblutspiegels zu erreichen und besteht dadurch ein erhöhtes Atheroskleroserisiko, sind zusätzlich lipidsenkende Medikamente indiziert. Durch die Behandlung mit lipidsenkenden Medikamenten kann eine deutliche Verminderung dieser Erkrankungen erzielt werden. In aktuellen Studien, z.B. LCAS - Lipoprotein and Coronary Arterosclerosis Study; LIPID - Long-term Intervention with Pravastatin in Ischemic Disease; CARE - The Cholesterol and Recurrent Events Trial, konnte gezeigt werden, daß die medikamentöse Therapie zur Prävention von arteriosklerotischen Gefäßerkrankungen auch dann greift, wenn die Blutfettwerte vor Behandlung nur leicht erhöht sind oder sogar im Normbereich liegen.

Der Langzeiterfolg von Bypass-Operationen wird oft durch Atherosklerose in den Bypässen begrenzt. Die Progression der Atherosklerose läßt sich durch eine konsequente Senkung des Blut-LDL-Spiegels reduzieren. Es konnte gezeigt werden, daß die Nachbehandlung mit Lovastatin Bypässe länger offen hält und somit zu einer verbesserten Prognose von Bypass-Operationen führt.

Lipidsenkende Medikamente lassen sich einteilen in Stoffe, die den Triglycerid- sowie den Cholesterol-Blutspiegel senken, und Substanzen, die hauptsächlich den Cholesterol-Blutspiegel senken. Zu der ersten Stoffgruppe gehören zum Beispiel Aryloxyalkancarbonsäuren, z.B. Clofibrat, Etofibrat, Etofyllinclofibrat, Bezafibrat, Fenofibrat, Gemfibrozil, Nicotinsäure, Nicotinylalkohol und Acipimox. Beispiele für Stoffe, die hauptsächlich den Cholesterol-Blutspiegel beeinflussen, sind: Anionenaustauscherharze wie Colestyramin oder Colestipol; Hemmstoffe der Hydroxymethyl-Glutaryl-CoA-Reduktase, HMG-CoA-Reduktase, Hemmstoffe wie Lovastatin, Simvastatin, Mevastatin, Pravastatin, Fluvastatin, Cerivastatin oder Atorvastatin, Probucol, Dextrothyroxin und Sitosterol.

Diese Stoffe hemmen die Hydroxy-methyl-Glutaryl-CoA-Reduktase, ein frühes Stadium der Cholesterolsynthese. Diese Inhibitoren sind die wirksamsten Substanzen zur Behandlung einer Hypercholesterolämie.

Die derzeit handelsüblichen Arzneiformen sind Tabletten und Kapseln in einer Dosierung von 5 bis 40 mg. Die wirkstoffe werden entweder in ihrer aktiven Form, das heißt als Natriumsalz der Hydroxysäure (z.B. Pravastatin), oder als Pro-Drug, daß heißt in ihrer Lactonform (z.B. Lovastatin), verabreicht. Nach oraler Gabe werden jedoch nur circa 30 % der applizierten Dosis aus dem Gastrointestinaltrakt absorbiert. Der absorbierte wirkstoffanteil unterliegt dann einem ausgeprägten First-Pass-Effekt. Die absolute Bioverfügbarkeit liegt im Bereich von 10 bis 30 %. Die durchschnittliche Eliminationshalbwertszeit der aktiven Wirkstoffform liegt im Bereich von 1-2 Stunden; Ausnahme ist Atorvastatin mit 14 h.

Stand der Technik sind Zubereitungen, die HMG-CoA-Reduktase-Hemmstoffe enthalten und zur topischen Anwendung bestimmt sind. Substanzen dieser Klasse können zur Therapie von Hauterkrankungen eingesetzt werden. Dabei dienen die HMG-CoA-Reduktase-Hemmstoffe als Antipsoriatika, beispielsweise als Hautalterungsschutzmittel, oder zur Behandlung von Akne. Dabei ist der Wirkstoff in einer klassischen Arzneiform wie Gel, Salbe oder Creme eingearbeitet. Ein nicht therapeutischer Einsatz besteht darin, die hier beschriebene Substanzklasse zur Steigerung der perkutanen Absorptionsrate von normalerweise nur ungenügend resorbierbaren wirkstoffen einzusetzen.

Systeme, die eine transdermalen Applikation dieser Substanzklasse in Betracht ziehen, sind seltener beschrieben.
US 5,629,014 beschreibt ein System, welches unter anderem zur kontrollierten Abgabe von Lovastatin an die Haut oder Schleimhäute geeignet ist. Dieses System umfaßt einen als Wirkstoffreservoir dienenden mikrozellularen Polyester bzw. Polyetherschaum. Da dieser Schaum selbst nicht klebend ist, ist ein zusätzliches Mittel zur Fixierung des Schaumes auf der Applikationsfläche notwendig. Dieses schaumartige System fällt relativ dick und inflexibel aus. Somit ist die Anwendung durch den Patienten nicht sehr praktikabel, da das System, durch seine Höhe exponiert, leicht ungewollt entfernbar ist und Körperbewegungen nicht mitmacht.

Eine transdermale Applikation von Lipidsenkern, als Gesamtgruppe genannt, wird in der DE 36 34 016 C2 erwähnt. Dieses System ist dadurch gekennzeichnet, daß die für die Haftung verantwortliche Komponente getrennt von dem nicht klebenden wirkstoffreservoir vorliegt.

EP-A-0 782 861 offenbart perkutan absorbierbare Zubereitungen, beispielsweise in Form eines Reservoirpflasters, die einen Arzneistoff und einen Lösungsvermittler enthalten. Bei dem Lösungsvermittler handelt es sich um eine Verbindung aus einer bestimmten Gruppe von N-substituierten O-Toluidinen. Diese Verbindungen bewirken außerdem eine Verbesserung der Wirkstoff-Absorption durch die Haut. Als Wirkstoffe werden unter anderen auch Lipidsenker wie Pravastatin, Lovastatin, Fluvastatin und Simvastatin in Betracht gezogen. Als Beispiele werden Hautpflaster beschrieben, die entzündungshemmende Wirkstoffe, kardiovaskuläre Wirkstoffe, Psychopharmaka oder Steroidhormone enthalten.

Ausgehend vom vorgenannten Stand der Technik liegt der Erfindung die Aufgabe zugrunde, eine Zubereitung, enthaltend mindestens einen die Blutfettwerte eines Organismus beeinflussenden Wirkung anzugeben, mit welcher eine über längere Zeiträume gleichbleibend langsame sowie exakt dosierbare Abgabe der therapeutisch wirksamen Substanz erzielt werden kann und bei der insbesondere die absolute Bioverfügbarkeit der Substanz bei anwenderfreundlicher Art der Applikation gewährleistet ist, uns wobei die Zubereitung als Wirkstoff-Reservoir dient.

Zur Lösung der Aufgabe wird bei einer Zubereitung der im Oberbegriff des Anspruch 1 genannten Art mit der Erfindung vorgeschlagen, daß der genannte Wirkstoff aus der Gruppe der Hydroxymethyl-Glutaryl-CoA-Reductase hemmenden wirkstoffe ausgewählt ist, daß die selbstklebende Matrixschicht eine Masse auf der Basis von Polyacrylat oder eine Masse auf Silikonbasis ist, und daß der genannte Hilfsstoff aus der Gruppe ausgewählt ist, die aus Pyrrolidonderivaten, Fettsäuren, Fettalkoholen, Fettsäure-Estern, Fett-Ethern, Paraffinderivaten, Terpenen, Ethylenglykolmonoalkylethern, Polyoxyethylenalkylethern, Polyoxyethylenarylethern, Polyoxyethylenalkylestern, Polyoxypropylenalkylethern, Propylenglykolfettsäurederivaten, Glycerinfettsäureestern, Polysorbaten, Poloxameren, Dialkylsulfoxiden, Harnstoff und Harnstoff-Derivaten, Glyzerin, nativen Ölen, Laurocapramen, Phospholipiden, Amiden, Aminosäuren, N,N-Dimethylformamid, N-Methylformamid, Acetoniden, Calciumthioglycolat, Propylenglycol, Polyethylenglycol, Alkylsulfaten, Natriumlaurylsulfat, Tetrahydrofurfurylalkohol, makrocyclischen Verbindungen oder polaren Lösungsmittel wie Panthenol besteht.

Das transdermale therapeutische Applikationssystem nach der Erfindung gewährleistet eine höchst effektive Arzneimitteltherapie, bei der die Freisetzung des Wirkstoffs über einen langen Zeitraum annähern konstant bleibt und in exakt kontrollierbarer Weise gelingt, wobei auch die absolute Bioverfügbarkeit der Substanz signifikant gesteigert wird.

Weitere Ausgestaltungen sind entsprechend den Unteransprüchen vorgesehen. Insbesondere ist die selbstklebende Masse dadurch gekennzeichnet, daß darin mindestens ein die Hydroxy-Methyl-Glutaryl-CoA-Reduktase hemmender Wirkstoff enthalten ist, und daß Strukturelemente einer Beta-Hydroxycarbonsäure (I) oder eines Tetrahydro-4-hydroxy-6-oxo-2H-pyrans (II) enthalten sind. Der Wirkstoff kann in Form seines Salzes oder in Form eines Esters vorliegen.

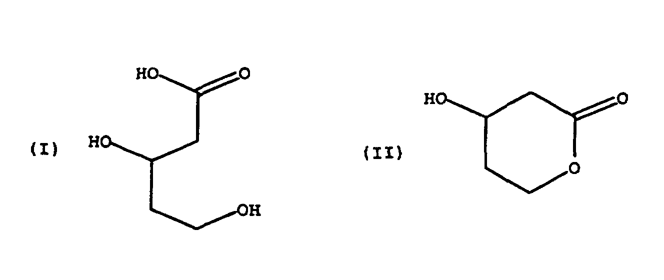

Für das erfindungsgemäße Pflaster kann eine selbstklebende Masse auf Basis von Polyacrylat oder eine Masse auf Silikonbasis eingesetzt werden.

Massen auf Polyacrylatbasis sind dadurch gekennzeichnet, daß zu ihrer Herstellung Acrylsäure und/oder Alkylacrylsäure, insbesondere Methacrylsäure bzw. dessen Derivate, insbesondere die Alkylester, eingesetzt werden. Unter den Alkylestern der Acrylsäure und/oder Methacrylsäure sind diejenigen mit 1 bis 18 Kohlenstoffatomen im Alkylrest bevorzugt, insbesondere Methyl-, Ethyl-, n-Butyl-, Isobutyl-, Pentyl-, 2-Ethylbutyl-, n-Hexyl-, Heptyl-, n-Octyl-, Isooctyl-, 2-Ethylhexyl-, n-Decyl-, Isodecyl-, n-Dodecyl- und Stearylacrylat bzw. -methacrylat. Daneben können weitere Comonomere am Aufbau des Polymers/Copolymers beteiligt sein. Beispiele sind Acryl- und/oder Methacrylamid, Hydroxyalkylester und Polyalkylenglykolester der Acryl- und/oder Methacrylsäure, stickstoffhaltige Monomere der Acryl- und/oder Methacrylsäure oder deren Salze, Ethylen, Vinylacetat, Vinylpropionat, Vinylbutyrat, Vinylpyrrolidon, Vinylchlorid, Vinyltoluol, Acrylnitril oder Styrol.

Massen auf Silikonbasis sind dadurch gekennzeichnet, daß sie ein großes freies Volumen, eine niedrige Glasübergangstemperatur, eine hohe Flexibilität, und eine hohe Gaspermeabilität aufweisen, biokompatibel sind, eine niedrige Oberflächenspannung und eine gute Benetzbarkeit besitzen, wärmestabil sowie chemisch inert sind und über gute Klebrigkeit, Adhäsion und Kohäsion verfügen. Typischerweise enthalten Massen auf Silikonbasis ein Polykondensat, umfassend ein niedrigviskoses Polydimethylsiloxan und ein Silikatharz, gekennzeichnet durch ein dreidimensionales Netzwerk. Zur Steigerung der sogenannten Aminresistenz kann die endständige Hydroxylgruppierung des Polydimethylsiloxans mit Trimethylsiloxan kondensiert sein.

Ferner kann eine mit der selbstklebenden Masse verbundene Rückschicht enthalten sein. Diese kann undurchlässig für den Wirkstoff sein und okklusiven Charakter aufweisen. Es können beliebige Materialien eingesetzt werden, die in herkömmlichen Präparaten Verwendung finden. Beispiele für derartige Materialien sind Celluloseacetat, Ethylcellulose, Polyethylenterephthalat, weichgemachte Vinylacetat-Vinylchlorid-Copolymerisate, Nylon, Ethylen-Vinylacetat-Copolymerisate, weichgemachtes Polyvinylchlorid, Polyurethan, Polyvinylidenchlorid, Polypropylen, Polyethylen, Polyamid oder Aluminium.

Die Zusammensetzung kann ferner enthalten: Klebrigmacher, Penetrationsverbesserer, Mittel zur Linderung von Hautreizungen, Metallionen wie Aluminium oder Titan, und zur Erhöhung der Kohäsion Weichmacher, Paraffine, zyklische Kohlenwasserstoffe oder pflanzlische Öle.

Als Mittel, welche die Klebrigkeit erhöhen, können Kolophoniumharze, Polyterpenharze, Petroleumharze, Cumaron-Inden-Harze, Terpenphenolharze, Kohlenwasserstoffharze oder flüssige Polybutenharze verwendet werden.

Als Mittel, welche die Penetration des Wirkstoffes verbessern, werden verwendet: Pyrrolidonderivate, Fettsäuren, Fettalkohole, Fettsäure-Ester, Fett-Ether, Paraffinderivate, Terpene, Ethylenglykolmonoalkylether, Polyoxyethylenalkylether, Polyoxyethylenarylether, Polyoxyethylenalkylester, Polyoxypropylenalkylether, Propylenglykolfettsäurederivate, Glycerinfettsäureester, Polysorbate, Poloxamere, Dialkylsulfoxide, Harnstoff und Harnstoff-Derivate, Glyzerin, native Öle, Laurocaprame, Phospholipide, Amide, Aminosäuren, N,N-Dimethylformamid, N-Methylformamid, Acetonide, Calciumthioglycolat, Propylenglycol, polyethylenglycol, Alkylsulfate, Natriumlaurylsulfat, Tetrahydrofurfurylalkohol, makrocyclische Verbindungen oder polare Lösungsmittel wie Panthenol.

Die erfindungsgemäße Zusammensetzung kann auch Mittel zur Linderung von Hautreizungen enthalten wie Bisabolol, Kamillenöl, Allantoin, Glyzerin oder Dipantenol.

Im folgenden wird die Erfindung anhand von Beispielen näher erläutert:

### BEISPIEL: 1

626 g einer Lösung eines selbstklebenden Polymers auf Silikonbasis (z.B. BIO PSA X7-4301, 70 Gew.-% in n-Heptan) und 48 g 2-Pyrrolidon (mit Lovastatin) wurden gemischt und als 600 µm dicker Film mit Hilfe einer Rakel auf eine fluorpolymerisierte Polyesterfolie (z.B. Scotchpak® 1022) aufgetragen. Der feuchte Film wurde für 30 Minuten bei 50°C getrocknet und anschließend mit einer Polyesterfolie (z.B. Hostaphan RN 15) kaschiert. Das Flächengewicht eines derartig hergestellten Klebefilms betrug etwa 300 g/m². Aus dem Laminat wurden mittels geeigneter Stanze TTS der gewünschten Größe ausgestanzt und die in-vitro Permeation durch isolierte Kuheuter-Haut gemessen. Die Flußrate lag über einen Zeitraum von 72 Stunden durchschnittlich bei 0,3 µg/cm²/h.

### BEISPIEL 2

459,2 g einer Lösung eines selbstklebenden Polymers auf Silikonbasis (z.B. BIO PSA X7-4301, 70 Gew.-% in n-Heptan) und 6,6 g Ethyloleat (mit Lovastatin) wurden gemischt und als 600 µm dicker Film mit Hilfe einer Rakel auf eine fluorpolymerisierte Polyesterfolie (z.B. Scotchpak® 1022) aufgetragen. Der feuchte Film wurde für 30 Minuten bei 50°C getrocknet und anschließend mit einer Polyesterfolie (z.B. Hostaphan RN 15) kaschiert. Das Flächengewicht eines derartig hergestellten Klebefilms betrug etwa 300 g/m². Aus dem Laminat wurden mittels geeigneter Stanze TTS der gewünschten Größe ausgestanzt und die in-vitro Permeation durch isolierte Kuheuter-Haut gemessen. Über einen Zeitraum von 72 Stunden diffundierte der eingearbeitete Wirkstoff nahezu quantitativ durch die Kuheuter-Haut.

### BEISPIEL 3

85,34 g eines selbstklebenden, Carboxylgruppen enthaltenden Polyacrylats (z.B. Durotak 387-2052, 48,1 Gew.-% in einem Gemisch aus Ethylacetat, n-Heptan, 2-Propanol und Ethanol), 85,34 g eines hydrophilen Acrylatklebstoffgemisches (z.B. Plastoid E 35 H, 60 Gew.-% in Ethylacetat), 12,5 g Ethylacetat sowie 8,4 g 2-Pyrrolidon (mit Lovastatin) wurden gemischt und als 400 um dicker Film mit Hilfe einer Rakel auf eine silikonisierte Polyesterfolie (z.B. Hostaphan® RN100) aufgetragen. Der feuchte Film wurde für 30 Minuten bei 50**°**C getrocknet und anschließend mit einer Polyesterfolie (z.B. Hostaphan RN 15) kaschiert. Das Flächengewicht eines derartig hergestellten Klebefilms betrug etwa 130 g/m².

## Patentansprüche

1. Zubereitung in Form eines transdermalen therapeutischen Systems, welches in einer an der hautabgewandten Seite mit einer wirkstoffundurchlässigen Rückschicht abdeckbaren selbstklebenden Matrixschicht mindestens einen die Blutfettwerte beeinflussenden Wirkstoff sowie mindestens einen die Permeation des Wirkstoffs durch die Haut förderndenden Hilfsstoff enthält, **dadurch gekennzeichnet, daß**
der genannte Wirkstoff aus der Gruppe der Hydroxymethyl-Glutaryl-CoA-Reductase hemmenden wirkstoffe ausgewählt ist,
die selbstklebende Matrixschicht eine Masse auf der Basis von Polyacrylat oder eine Masse auf Silikonbasis ist, und
der genannte Hilfsstoff aus der Gruppe ausgewählt ist, die aus Pyrrolidonderivaten, Fettsäuren, Fettalkoholen, Fettsäure-Estern, Fett-Ethern, Paraffinderivaten, Terpenen, Ethylenglykolmonoalkylethern, Polyoxyethylenalkylethern, Polyoxyethylenarylethern, Polyoxyethylenalkylestern, Polyoxypropylenalkylethern, Propylenglykolfettsäurederivaten, Glycerinfettsäureestern, Polysorbaten, Poloxameren, Dialkylsulfoxiden, Harnstoff und Harnstoff-Derivaten, Glyzerin, nativen Ölen, Laurocapramen, Phospholipiden, Amiden, Aminosäuren, N,N-Dimethylformamid, N-Methylformamid, Acetoniden, Calciumthioglycolat, Propylenglycol, Polyethylenglycol, Alkylsulfaten, Natriumlaurylsulfat, Tetrahydrofurfurylalkohol, makrocyclischen Verbindungen oder polaren Lösungsmittel wie Panthenol besteht.

2. Zubereitung nach Anspruch 1, **dadurch gekennzeichnet, daß** der die Hydroxymethyl-Glutaryl-CoA-Reduktase hemmende Wirkstoff die Strukturmerkmale einer Beta-Hydroxycarbonsäure oder eines Tetrahydro-4-hydroxy-6-oxo-2H-pyrans im Molekül enthält.

3. Zubereitung nach Anspruch 1 oder 2, **dadurch gekennzeichnet, daß** der die Hydroxymethyl-Glutaryl-CoA-Reduktase hemmende Wirkstoff in Form eines Salzes oder in Form eines Esters vorliegt.

4. Zubereitung nach einem oder mehreren der Ansprüche 1 bis 3, **dadurch gekennzeichnet, daß** der die Hydroxymethyl-Glutaryl-CoA-Reduktase hemmende Wirkstoff Lovastatin, Simvastatin, Mevastatin, Pravastatin, Fluvastatin, Atorvastatin, Eptastatin oder Cerivastatin ist.

5. Zubereitung nach einem oder mehreren der Ansprüche 1 bis 4, **dadurch gekennzeichnet, daß** die selbstklebende Schicht der Matrix mindestens ein Homo-, Co- oder Blockpolymer enthält.

6. Zubereitung nach einem oder mehreren der Ansprüche 1 bis 5, **dadurch gekennzeichnet, daß** die selbstklebende Masse ein Schmelzkleber ist.

7. Zubereitung nach einem oder mehreren der Ansprüche 1 bis 6, **dadurch gekennzeichnet, daß** die selbstklebende Masse mindestens einen die Klebrigkeit erhöhenden Hilfsstoff enthält.

8. Zubereitung nach einem oder mehreren der Ansprüche 1 bis 7, **dadurch gekennzeichnet, daß** die selbstklebende Masse mindestens einen Weichmacher enthält.

9. Zubereitung nach einem oder mehreren der Ansprüche 1 bis 8, **dadurch gekennzeichnet, daß** die selbstklebende Masse mindestens einen Hautreizungen lindernden Hilfsstoff enthält.

10. Zubereitung nach einem oder mehreren der Ansprüche 1 bis 9, **dadurch gekennzeichnet, daß** die selbstklebende Masse mindestens eine die Kohäsion beeinflussenden Stoff enthält.

11. Verwendung der Zubereitung nach einem der Ansprüche 1 bis 10 zur Herstellung eines Mittels zum Senken erhöhter Plasmalipidspiegel, insbesondere bei systemischen Lipidstoffwechselstörungen, sogenannten Hyperlipoproteinämien, und Gefäßerkrankungen wie Herzinfarkt sowie bei arterieller Verschlußerkrankung.

## Claims

1. Preparation in the form of a transdermal therapeutic system containing at least one active substance which has an influence on the lipid blood levels and at least one auxiliary substance enhancing the permeation of the active substance through the skin in a self-adhesive matrix layer which can be covered at the side facing away from the skin with an active substance-impermeable backing layer, **characterized in that**
the said active substance is selected from the group of active substances inhibiting hydroxymethylglutaryl-CoA reductase,
the self-adhesive matrix layer is a mass based on polyacrylate, or a mass based on silicone, and that
the said auxiliary substance is selected from the group consisting of pyrrolidone derivatives, fatty acids, fatty alcohols, fatty acid esters, fatty ethers, paraffin derivatives, terpenes, ethylene glycol monoalkyl ethers, polyoxyethylene alkyl ethers, polyoxyethylene aryl ethers, polyoxyethylene alkyl esters, polyoxypropylene alkyl ethers, propylene glycol fatty acid derivatives, glycerol fatty acid esters, polysorbates, poloxamers, dialkyl sulfoxides, urea and urea derivatives, glycerol, native oils, laurocaprames, phospholipides, amides, amino acids, N,N-dimethyl formamide, N-methyl formamide, acetonides, calcium thioglycolate, propylene glycol, polyethylene glycol, alkyl sulfate, sodium lauryl sulfate, tetrahydrofurfuryl alcohol, macrocyclic compounds or polar solvents such as panthenol.

2. Preparation according to claim 1, **characterized in that** the active substance inhibiting hydroxymethylglutaryl-CoA reductase contains the structural features of a beta-hydroxy-carboxylic acid or of a tetrahydro-4-hydroxy-6-oxo-2H-pyrans in its molecule.

3. Preparation according to claim 1 or 2, **characterized in that** the active substance inhibiting hydroxymethylglutaryl-CoA reductase is present in the form of a salt or in the form of an ester.

4. Preparation according to one or more of claims 1 to 3, **characterized in that** the active substance inhibiting hydroxymethylglutaryl-CoA reductase is lovastatin, simvastatin, mevastatin, pravastatin, fluvastatin, atorvastatin, eptastatin or cerivastatin.

5. Preparation according to one or more of claims 1 to 4, **characterized in that** the self-adhesive layer of the matrix contains at least one homo-, co- or block polymer.

6. Preparation according to one or more of claims 1 to 5, **characterized in that** the self-adhesive mass is a hot-melt adhesive.

7. Preparation according to one or more of claims 1 to 6, **characterized in that** the self-adhesive mass contains an auxiliary substance which increases tack.

8. Preparation according to one or more of claims 1 to 7 **characterized in that** the self-adhesive mass contains at least one plasticizer.

9. Preparation according to one or more of claims 1 to 8, **characterized in that** the self-adhesive mass contains at least one auxiliary substance alleviating skin irritations.

10. Preparation according to one or more of claims 1 to 9, **characterized in that** the self-adhesive mass contains at least one substance having an influence on cohesion.

11. The use of the preparation according to any one of claims 1 to 10 for producing a means for lowering increased plasma lipid levels, especially in the case of systemic lipid metabolism disturbances, so-called hyperlipoproteinemias, and vascular diseases such as cardiac infarction, as well as in the case of occlusive arterial disease.

## Revendications

1. Préparation sous la forme d'un système thérapeutique transdermique, qui contient, dans une couche matricielle auto-adhésive qui peut être recouverte, sur son côté qui se détourne de la peau, d'une couche dorsale imperméable aux substances actives, au moins une substance active influençant la teneur du sang en matières grasses, ainsi qu'au moins un adjuvant favorisant la perméation de la substance active à travers la peau, **caractérisée en ce que**
la substance active mentionnée est choisie parmi le groupe des substances actives inhibant l'hydroxyméthyl-glutaryl-CoA-réductase,
la couche matricielle auto-adhésive est une matière à base de polyacrylate ou une matière à base de silicone, et
l'adjuvant mentionné est choisi parmi le groupe qui est constitué par des dérivés de la pyrrolidone, des acides gras, des alcools gras, des esters d'acides gras, des éthers gras, des dérivés de paraffine, des terpènes, des éthers monoalkyliques d'éthylèneglycol, des éthers alkyliques de polyoxyéthylène, des éthers aryliques de polyoxyéthylène, des esters alkyliques de polyoxyéthylène, des éthers alkyliques de polyoxypropylène, des dérivés d'acides gras de propylèneglycol, des esters d'acides gras de glycérol, des polysorbates, des poloxamères, des dialkylsulfoxydes, l'urée et des dérivés de l'urée, le glycérol, des huiles naturelles, des laurocaprames, des phospholipides, des amides, des aminoacides, le N,N-diméthyl-formamide, le N-méthylformamide, des acétonides, le thioglycollate de calcium, le propylèneglycol, le polyéthylèneglycol, des sulfates d'alkyle, le laurylsulfate de sodium, l'alcool tétrahydrofurfurylique, des composés macrocycliques ou encore des solvants polaires tels que le panthénol.

2. Préparation selon la revendication 1, **caractérisée en ce que** la substance active inhibant l'hydroxyméthyl-glutaryl-CoA-réductase contient, dans sa molécule, les caractéristiques de structure d'un acide bêta-hydroxycarboxylique ou d'un tétrahydro-4-hydroxy-6-oxo-2H-pyrane.

3. Préparation selon la revendication 1 ou 2, **caractérisée en ce que** la substance active inhibant l'hydroxyméthyl-glutaryl-CoA-réductase est présente sous la forme d'un sel ou sous la forme d'un ester.

4. Préparation selon une ou plusieurs des revendications 1 à 3, **caractérisée en ce que** la substance active inhibant l'hydroxyméthyl-glutaryl-CoA-réductase est la lovastatine, la simvastatine, la mévastatine, la pravastatine, la fluvastatine, l'atorvastatine, l'eptastatine ou la cérivastatine.

5. Préparation selon une ou plusieurs des revendications 1 à 4, **caractérisée en ce que** la couche auto-adhésive de la matrice contient au moins un homopolymère, un copolymère ou un polymère séquence.

6. Préparation selon une ou plusieurs des revendications 1 à 5, **caractérisée en ce que** la matière auto-adhésive est une colle thermofusible.

7. Préparation selon une ou plusieurs des revendications 1 à 6, **caractérisée en ce que** la matière auto-adhésive contient au moins un adjuvant qui augmente l'adhésivité.

8. Préparation selon une ou plusieurs des revendications 1 à 7, **caractérisée en ce que** la matière auto-adhésive contient au moins un plastifiant.

9. Préparation selon une ou plusieurs des revendications 1 à 8, **caractérisée en ce que** la matière auto-adhésive contient au moins un adjuvant qui atténue les irritations cutanées.

10. Préparation selon une ou plusieurs des revendications 1 à 9, **caractérisée en ce que** la matière auto-adhésive contient au moins une substance influençant la cohésion.

11. Utilisation de la préparation selon l'une quelconque des revendications 1 à 10, pour la production d'un agent destiné à abaisser un taux élevé de lipides plasmatiques, en particulier dans le cas de dysfonctionnements systémiques du métabolisme lipidique, ce que l'on appelle des hyperlipoprotéinémies, et dans le cas de maladies vasculaires telles que l'infarctus du myocarde, et également dans le cas d'une maladie artérielle oblitérante.
